# EUROPÄISCHE PATENTSCHRIFT

(11) **EP 1 872 372 B1**
(45) Veröffentlichungstag und Bekanntmachung des Hinweises auf die Patenterteilung: **02.12.2009**
(21) Anmeldenummer: 06723362.7
(22) Anmeldetag: 10.03.2006
(51) Int. Cl.: G21K 1/087, H01J 37/12, H05H 15/00, A61N 5/10

(54) **LASERBESTRAHLTER HOHLZYLINDER ALS LINSE FÜR IONENSTRAHLEN**
LASER IRRADIATED HOLLOW CYLINDER SERVING AS A LENS FOR ION BEAMS
CYLINDRE CREUX SOUMIS A L'ACTION D'UN RAYONNEMENT LASER, UTILISE EN TANT QUE LENTILLE POUR FAISCEAUX D'IONS

(30) Priorität: 16.03.2005 DE 102005012059
(43) Veröffentlichungstag der Anmeldung: 02.01.2008
(73) Patentinhaber: Heinrich-Heine-Universität Düsseldorf, 40225 Düsseldorf (DE); Centre National de la Recherche Scientifique, 75794 Paris Cedex 16 (FR); Ecole Polytechnique, 91128 Palaiseau Cedex (FR); The Queens University of Belfast, Belfast BT7 1NN (GB); Université Pierre et Marie Curie, 75005 Paris (FR)
(72) Erfinder: WILLI, Oswald, 40591 Düsseldorf (DE); FUCHS, Julien, F-75013 Paris (FR); BORGHESI, Marco, Belfast BT60HF, Northern Ireland (GB); TONCIAN, Toma, 40591 Düsseldorf (DE)
(74) Vertreter: Braun-Dullaeus, Karl-Ulrich
(86) Internationale Anmeldenummer: PCT/EP2006/002249
(87) Internationale Veröffentlichungsnummer: WO 2006/097252

(56) Entgegenhaltungen:
- EP-A- 0 225 717
- US-A- 5 382 866
- GABOR D: "A space-charge lens for the focusing of ion beams" NATURE, Bd. 160, Nr. 4055, 19. Juli 1947 (1947-07-19), Seiten 89-90, XP009068124 ISSN: 0028-0836
- GOVIL R ET AL: "UV laser ionization and electron beam diagnostics for plasma lenses" PARTICLE ACCELERATOR CONFERENCE, 1995., PROCEEDINGS OF THE 1995 DALLAS, TX, USA 1-5 MAY 1995, NEW YORK, NY, USA,IEEE, US, Bd. 2, 1. Mai 1995 (1995-05-01), Seiten 776-778, XP010165774 ISBN: 0-7803-2934-1
- MACKINNON A J ET AL.: "Proton radiography as an electromagnetic field and density perturbation diagnostic" REVIEW OF SCIENTIFIC INSTRUMENTS, Bd. 75, Nr. 10, Oktober 2004 (2004-10), Seiten 3531-3536, XP002387021
- ROTH M ET AL.: "Energetic ions generated by laser pulses: A detailed study on target properties" PHYSICAL REVIEW SPECIAL TOPICS - ACCELERATORS AND BEAMS, Bd. 5, 4. Juni 2002 (2002-06-04), Seiten 061301-1-061301-8, XP002387022
- TONCIAN T; BORGHESI M; FUCHS J; D'HUMIERES E; ANTICI P; AUDEBERT P; BRAMBRINK E; CECCHETTI C A; PIPAHL A; ROMAGNANI L; WILLI O: "Ultrafast Laser-Driven Microlens to Focus and Energy-Select Mega-Electron Volt Protons" SCIENCE, Bd. 312, Nr. 5772, 21. April 2006 (2006-04-21), Seiten 410-413, XP002387023

## Beschreibung

Die vorliegende Erfindung betrifft ein System zur Fokussierung und/oder zur Kollimierung eines Ionenstrahles, insbesondere eines Strahles hochenergetischer Protonen, wobei das System zunächst eine Linse mit einem für den Durchtritt des lonenstrahls geeigneten Linsenkörper aufweist, wobei außerdem Mittel zur Erzeugung eines insbesondere elektrostatischen Feldes vorgesehen sind, wobei das Feld sich innerhalb des Linsenkörpers ausbreitet und den Ionenstrahl fokussiert und/oder kollimiert. Die Erfindung betrifft gleichfalls ein Verfahren zur Fokussierung und/oder Kollimierung des Ionenstrahles.

Es ist bekannt, hochenergetische Ionenstrahlen beispielsweise mit elektrischen oder magnetischen Quadrupollinsen zu fokussieren. Da Quadrupole nur in einer Ebene fokussieren, während sie in der anderen Ebene defokussieren, werden sie im Strahlengang meist im Multiplett hintereinander angeordnet. Die fokussierende Wirkung der Quadrupollinsen wird dabei durch das Feld limitiert, das am Polschuh erzeugt werden kann. Neben Quadrupollinsen sind auch andere elektrische oder magnetische Linsen mit Rotationssymmetrie bekannt. Alle bekannten Systeme sind baulich verhältnismäßig aufwendig und für Ionenstrahlen mit einer großen energetischen Bandbreite, wie es bei laserinduzierten Protonenstrahlen der Fall ist, kaum geeignet. Dabei werden diese laserinduzierten Protonenstrahlen durch Bestrahlung einer dünnen Folie mit ultrakurzen (fs-Bereich) und hochintensiven (~10¹⁹ Watt) Laserpulsen erzeugt.

Aus dem Stand der Technik nach GABOR D: "A space-charge lens for the focusing of ion beams" NATURE, Bd. 160, Nr. 4055, 19. Juli 1947 (1947-07-19), Seiten 89-90, XP009068124 ISSN: 0028-0836, sind außerdem "Gabor"- (Plasma)-Linsen bekannt, die bezüglich ihrer optischen Eigenschaften mit Draht-Linsen zu vergleichen sind, wobei hochenergetische Ionenstrahlen durch ein azimuthales magnetisches Feld fokussiert werden, das durch einen axialen, durch Plasmaentladung erzeugten Strom hervorgerufen wird. Diese Plasmaentladung wird mit Elektroden erzeugt, die innerhalb des Linsenkörpers angeordnet sind. Plasmalinsen sind überaus aufwendig und finden daher nahezu ausschließlich in Forschungseinrichtungen der Schwerionen- und Hochenergiephysik Anwendung. Für den praktischen Einsatz, beispielsweise in medizinischen Therapiegeräten, sind Plasmalinsen kaum geeignet. Raumladungs- respektive Plasmalinsen sind zudem aus US-A-5 382 866 und EP-A-0 225 717 bekannt.

Zudem sind lasergenerierte Teilchenlinsen bekannt. Dabei geht GOVIL R ET AL: "UV laser ionization and electron beam diagnostics for plasma lenses" PARTICLE ACCELERATOR CONFERENCE, 1995., PROCEEDINGS OF THE 1995 DALLAS, TX, USA 1-5 May 1995, New York, NY, USA, IEEE, US, Bd. 2, 1. Mai 1995 (1995-05-01), Seiten 776-778, XP010165774 ISBN: 0-7803-2934-1 von der Einwirkung des Lasers auf ein bereits gasförmiges Plasma aus. MACKINNON A J ET AL: "Proton radiography as an electromagnetic field and density perturbation diagnostic" REVIEW OF SCIENTIFIC INSTRUMENTS, Bd. 75, Nr. 10, Oktober 2004 (2004-10), Seiten 3531-3536, XP002387021 nutzt die defokussierende / dekollimierende Linsenwirkung eines im Strahlengang erzeugten Plasmas von einem seitlich des Protonenstrahls angeordneten Drahtes zur Analyse des Plasmas selbst. In ROTH ET AL.: "Energetic ions generated by laser pulses: A detailed study on target properties" PHYSICAL REVIEW SPECIAL TOPICS - ACCELERATORS AND BEAMS, Bd. 5, 4. Juni 2002 (2002-06-04), Seiten 061301-1-061301-8, XP002387022 wird vorgeschlagen, zur Kollimation des Strahls die Form des zur Erzeugung des Protonenstrahls verwendeten Substrats anzupassen.

Aufgabe der Erfindung ist es nunmehr, ein System zu schaffen, das komfortabel handhabbar ist, sich mit einfachen Mitteln kostengünstig umsetzen lässt und das hervorragende fokussierende und/oder kollimierende Eigenschaften für hochenergetische Ionenstrahlen auch großer energetischer Bandbreite hat.

Zudem ist es die Aufgabe, ein einfaches Verfahren Fokussierung und/oder zur Kollimierung hochenergetischer Ionenstrahlen zu schaffen.

Diese Aufgaben werden durch das System nach Anspruch 1 und das Verfahren nach Anspruch 10 gelöst. Besonders vorteilhafte Ausführungsformen sind in den jeweiligen Unteransprüchen genannt.

Der Grundgedanke der Erfindung liegt darin, durch eine äußere Beaufschlagung des Linsenkörpers mit elektromagnetischer Strahlung innerhalb des Linsenkörpers ein insbesondere elektrostatisches Feld auszubilden, dessen Feldlinienverlauf eine kollimierende und/oder fokussierende Wirkung auf den hindurchtretenden Ionenstrahl hat. Dabei sei schon an dieser Stelle angemerkt, dass die Ausbildung eines solchen Feldes innerhalb eines vorteilhafterweise aus leitendem Material bestehenden hohlen Linsenkörpers unter bestimmten Umständen möglich ist, auch wenn dieses Phänomen der vom Faradayschen Käfig bekannten Gesetzmäßigkeit der völligen Feldlosigkeit im Inneren des leitenden Körpers widerspricht. Dieser Effekt wird, wenn auch in weit geringerem Maß, gleichsam bei Bestrahlung von isolierendem Material beobachtet.

Die Ausbildung des Feldes innerhalb des Linsenkörpers beruht auf dem oben angesprochenen Effekt, der auch für die laserinduzierten Protonenstrahlen verantwortlich ist. Dabei ist dem Feld im wesentlichen elektrostatische Natur zuzuschreiben. So werden bei der Wechselwirkung von intensiven Laserpulsen mit einer dünnen, insbesondere aus leitendem Metall bestehenden, Folie gepulste Elektronen- und Protonenstrahlen mit hoher Energie und Dichte in und/oder an der Oberfläche der Folie erzeugt. Diese Plasmaelektronen werden senkrecht zur Folienoberfläche beschleunigt, wobei sich starke Raumladungen ausbilden, da die um ein Vielfaches schwereren Protonen nicht folgen können. Auf der Rückseite der Folie treten somit hohe Feldstärken von bis zu 10¹² V/m auf. Das bedeutet im Falle eines aus einer solchen Folie gefertigten Hohlkörpers, dass sich an seiner Innenwand Elektronenwolken ausbilden und sich die Ladung über die innere Oberfläche verschiebt, wobei sich innerhalb des Zylinders ein Feld ausgebildet, das in diesem Fall radial ausgerichtet ist. Dieses elektrische Feld hat auf den durch den Zylinder hindurchtretenden Ionenstrahl eine entsprechende Wirkung, die im Fall des radialen Feldes fokussierend respektive kollimierend ist.

Die Tatsache, dass mit Hochintensitätslaserpulsen bestrahlte Targets, wegen der Erzeugung von lasergetriebenen heißen Elektronen eine kurzzeitige positive Aufladung erfahren, wird durch Experimente bestätigt. Eine Abschätzung anhand experimenteller Daten zeigt, dass eine Ladung Q von etwa 2,5 10⁻⁸C und ein elektrisches Feld E von ungefähr 10¹⁰ V/m entstehen kann. Das mit einer solchen Aufladung verbundene elektrische Feld ist ohne weiteres im Stande, Protonen einer Energie von einigen MeV abzulenken.

Inwieweit sich dieser Effekt ausbildet häng von der Dicke der Wandung und der Qualität, insbesondere der Energiedichte, der elektromagnetischen Strahlung ab. Diese Parameter müssen derart eingestellt werden, dass der Strahl, wie oben beschrieben, in der Wandung freie Elektronen erzeugt, die ausreichende Energie haben, um die Wandung zu durchdringen und eine Elektronenwolke an der Innenseite der Wandung auszubilden. Für diesen Effekt spielt die Geometrie des Linsenkörpers zunächst keine wesentliche Rolle. So braucht der Linsenkörper auch nicht zwangsläufig eine geschlossene Mantelfläche aufzuweisen. Allerdings ist darauf zu achten, dass die Möglichkeit einer homogenen Ladungsverteilung in der Wandung gegeben ist. Es sollte sich daher um eine mehr oder weniger geschlossene Fläche handeln. Da die Gestaltung des Linsenkörpers Einfluss auf die Form des Feldes hat, ist eine axialsymmetrische Form zu bevorzugen.

Um den erfindungsgemäß auszunutzenden Effekt zu erzielen, bedarf es, wie gesagt, einerseits eines Linsenkörpers mit dünner Wand, wobei im Falle von Laserintensitäten von etwa 10¹⁹ W/cm² Wandstärken zwischen etwa 0,01 mm und 0,1 mm als dünnwandig angesehen werden. Dabei definiert sich die mögliche Grenzdicke der Wandung über die Qualität der elektromagnetischen Strahlung, die innerhalb des Materials den beschriebenen Effekt erzeugen muss. Der gewünschte Effekt kann mit Laserpulsen einer Intensität von etwa 10¹⁹ W/cm² erreicht werden, die auf eine etwa 50 µm starke Ni-Folie treffen.

Zum Erhalt einer besonders gut fokussierenden Wirkung ist es vorteilhaft, wenn als Linsenkörper ein Hohlzylinder mit kreisförmigem Querschnitt eingesetzt wird, wobei die Bestrahlung der Außenwand mit einem hochintensiven Kurzimpulslaser erfolgt, die eine vorübergehende Aufladung erzeugt. Innerhalb eines solchen Holzylinders bildet sich ein radiales Feld aus. Ein fokussierender Effekt wird z.B. beobachtet, wenn der Zylinder, der seitlich von dem Laser bestrahlt wird, einen Durchmesser von etwa 1 mm aufweiset. Durch die Bestrahlung entsteht eine transiente positive Aufladung des Zylinders und somit das elektrische Feld, das dessen Feldlinien senkrecht auf die Zylinderoberfläche stehen. Durch dieses elektrische Feld werden die Ionen, insbesondere hochenergetische Protonen, in Richtung der Zylinderachse abgelenkt und damit fokussiert.

Generell lässt sich die erfindungsgemäße Art der Fokussierung auf beliebige Ionenstrahlen aus beliebigen Quellen anwenden, wobei die Ionen auch auf beliebige Weise eine Beschleunigung erfahren können. In einer vorteilhaften Ausführungsform ist das erfindungsgemäße System jedoch zusätzlich mit einer Ionenquelle ausgestattet, die ohne eine separate Beschleunigungsstrecke und ohne weiteren baulichen Aufwand beschleunigte Ionen erzeugt. Als Quelle für Protonen bieten sich wiederum laserinduzierte Protonenstrahlen an, da ein entsprechender Laserstrahl, wie oben beschrieben, ehedem zur Verfügung steht.
Mit einer solchen Ionenquelle lässt sich ein kompaktes System konzipieren, das komfortabel handhabbar ist und sich daher für den Einsatz in der medizinischen Therapie eignet. Auch wenn es möglich ist, zwei getrennte aber synchronisierte Laser zu nutzen, ist es doch vorteilhaft, im System Mittel vorzusehen, die den Strahl eines einzigen gepulsten Lasers teilen. Dabei werden mit einem Teilstrahl die Protonen erzeugt und mit dem anderen Teilstrahl die Linse aufgeladen. Um eine Energieselektion der Protonen vornehmen zu können, ist es vorteilhaft eine insbesondere variable Verzögerung des die Linse beaufschlagenden Strahlanteiles gegenüber dem protonenerzeugenden Strahlanteil vorzusehen.

Mit dem erfindungsgemäßen System ist neben der Fokussierung und Kollimierung auch die Energieselektierung möglich, so dass der Protonenstrahl für die eine Vielzahl von Anwendungen angepasst werden kann. Damit bildet die Erfindung ein wichtiges und flexibles Zwischenwerkzeug, um den Protonenstrahl in eine geeignete Form für eine bestimmte Anwendung bringen zu können. Dabei hängt das selektive Fokussieren bestimmter Energien von der Lebensdauer des elektrostatischen Feldes ab, das nach einer gewissen Zeit entweder durch nachfließende Ladung oder durch Rekombination der Ladungsträger zusammenbricht. Die Geschwindigkeit, mit der die Ladung nachfließen kann steht dabei in Abhängigkeit von der Aufhängung des Linsenkörpers. So bleiben die Felder innerhalb des Linsenkörpers bei einer möglichst gut elektrisch isolierten Aufhängung, beispielsweise an sehr dünnen Drähten und im Vakuum, für etwa 100 ps bestehen. Dabei gilt: Je schneller die Entladung geschieht, desto selektiver fokussiert die Linse eine bestimmte Energie.

Generell gibt es neben der Medizin, insbesondere der Tumortherapie, vielfältige Anwendungen der Protonen- und Ionenbeschleunigung durch ultrakurze, intensive Laserpulse. Zu nennen sind beispielsweise die innertiale Fusionsenergie, das isochore Heizen von Materie, die Protonenlithographie, die Protonenradiographie, die Untersuchung innerer Strukturen von statischen oder dynamischen Systemen sowie die Detektion von elektrischen und/oder magnetischen Feldern, die während der Interaktion intensiver Laserpulse mit hochdichter Materie gebildet werden.

Wie schon beschrieben, entstehen die hochenergetischen Protonen bei der Wechselwirkung zwischen dem Hochintensitätslaserpuls und der dichten Materie, wobei Leistungsdichten von größer 10¹⁹ W/cm² erreicht werden. Dabei treten die Protonen in einem kollimierten Strahl, mit einigen MeV Energie und mit relativ geringer Divergenz aus der Rückseite der Folie aus. Die Beschleunigung erfolgte innerhalb der laserbestrahlten dünnen Folie. Protonenstrahlen entstehen selbst dann, wenn das Target nominal keinen Wasserstoff enthält. Der Protonenstrahl hat einzigartige Eigenschaften, nämlich eine kleine Quellgröße, eine geringe Divergenz, eine kurze Dauer und eine große Dichte von bis zu 10¹² Protonen mit einigen Mev Energie. Im Vergleich zu herkömmlichen Quellen ist eine solche Quelle damit besonders vorteilhaft. Allerdings decken die so erzeugten Protonenstrahlen eine große Energiebandbreite ab.

Dabei weisen theoretische Modelle darauf hin, dass die Protonen ihre Energie aus dem enormen elektrischen Feld von bis zu einem MeV pro Mikrometer beziehen, das von den laserbeschleunigten, schnellen Elektronen über die Raumladung an der hinteren Targetoberfläche gebildet wird. Aus Simulationen ergibt sich, dass die Ladung des Protonenstrahls durch eine sich mitbewegende Wolke von Elektronen neutralisiert wird. Gemäß den Modellen werden die Protonen entsprechend der Laserpulsdauer von kleiner 1 ps beschleunigt. Es erscheint somit nicht möglich, einen solchen laserinduzierten Protonenstrahl mit anderen Mitteln als dem erfindungsgemäßen System zu fokussieren.

Ein weiterer Vorteil des erfindungsgemäßen Systems ist die verhältnismäßig hohe Wiederholungsrate, mit der sich die fokussierten Protonenpulse erzeugen lassen. Diese hängt lediglich vom Potential des verwendeten Lasers ab, wobei heutzutage je nach Leistung schon Wiederholungsraten von 10 Hz möglich sind. Damit wird das System noch interessanter für insbesondere medizinische Anwendungen.

Nachfolgend wird die Erfindung anhand der Figuren 1 bis 5 näher erklärt. Dabei zeigen:
- **Figur 1**: das Schema der Erzeugung eines laserinduzierten Protonenstrahls,
- **Figur 2**: eine Linse im Strahlengang des Protonenstrahls,
- **Figur 3**: Divergenzen untersuchter Protonenstrahlen,
- **Figur 4**: das Ergebnis einer Simulation und
- **Figur 5**: das Ergebnis einer Messung.

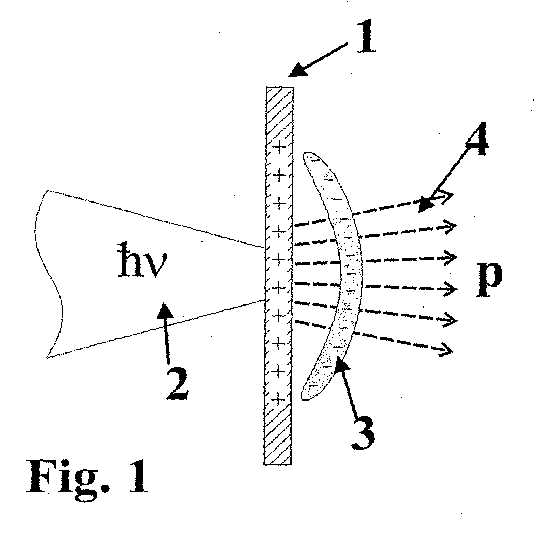

Figur 1 zeigt eine etwa 50 µm starke Al-Folie 1, die von einem kurzen Laserpuls 2 getroffen wird. Bei entsprechender Laserleistung werden die Elektronen in der Folie 1 mitgerissen und treten auf der Rückseite der Folie 1 aus. Dort bilden sie eine negativ geladene Elektronenwolke 3, wobei in der Folie entsprechend eine positive Ladung verbleibt. Diese für die Pulsdauer Zeit vorhandene Raumladung beschleunigt verbleibende Protonen (p) in der Folie, wobei die Protonen als Strahl 4 auf der Rückseite der Folie austreten.

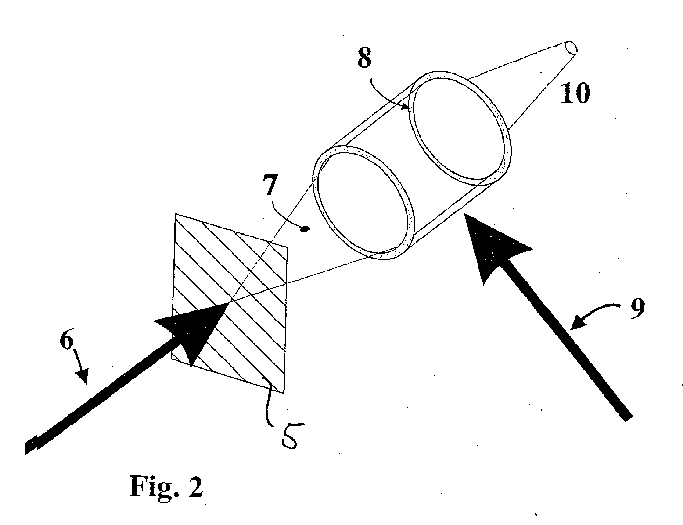

Figur 2 zeigt ein erfindungsgemäßes System zur Fokussierung eines mittels Laser erzeugten Protonenstrahls. Mit einer Anordnung, wie sie zu Figur 1 beschrieben ist, wird durch einen auf eine dünne Folie 5 gerichteten Laserpuls 6 mit einer Leistung von etwa 10¹⁹ W/cm² und einer Pulsdauer zwischen etwa 40 fs und einigen Pikosekunden ein Protonenstrahl 7 mit einer Divergenz zwischen 10° und 20° erzeugt. Das System hat als zentrales Element eine Linse mit einem für den Protonenstrahl 7 durchgängigen Linsenkörper in Form eines Hohlzylinders 8, der aus Aluminium einer Stärke zwischen 25 µm und 50 µm geformt ist. Der Hohlzylinder 8 hat eine Länge von etwa 1 mm bis 3 mm und einen Durchmesser zwischen 0,5 mm und 1,3 mm.

Als Mittel zur Erzeugung eines elektrischen Feldes innerhalb des Hohlzylinders ist ein weiterer Laserstrahl 9 vorgesehen, der auf die Außenseite der Wandung des Hohlzylinders 8 gerichtet ist. In diesem Fall stammt der Laserstrahl 9 und der Laserpuls 6 von derselben Quelle und haben daher dieselbe Pulscharakteristik. Es handelt sich um einen zeitverzögerten Teilstrahl desselben nicht dargestellten Lasers. Das durch den Laserpuls 9 in dem Hohlzylinder 8 erzeugte elektrische Feld fokussiert den divergenten Protonenstrahl 7 zu dem Protonenstrahl 10. Der eingehende Protonenstrahl 7 erfährt den fokussierenden Effekt und verlässt den Zylinder als ein kollimierter Teilstrahl 10 mit einem Querschnitt, der kleiner als der Zylinderradius ist. Der hinter dem Hohlzylinder 8 gemessene Protonenfluss ist um einen Faktor drei bis neun höher als im unfokussierten Fall, was zeigt, dass alle in den Zylinder eintretenden Protonen fokussiert werden.

Da das in dem Zylinder erzeugte elektrische Feld entsprechend der Pulsdauer des Laserstrahles 6 nur von kurzer Dauer ist, werden nur die Protonen kollimiert, die den Hohlzylinder 8 während seiner Aufladung passieren. Da diese Protonen eine gewisse Zeit für den Weg zwischen der Folie und dem Hohlzylinder 8 benötigten kollimiert das System die Protonen einer bestimmten Energie aus dem breitem Energiespektrum des ursprünglichen Protonenstrahls selektiv. Zudem ist es vorteilhaft, wenn am Ausgang eine Blende angeordnet ist, die nur den kollimierten Teil des Strahls passieren lässt. So wird es möglich, die Energie des einfallenden Protonenstrahls 7 für die jeweilige Anwendungen, z. B. die Protonentherapie zur Krebsbehandlung, individuell zuzuschneiden.

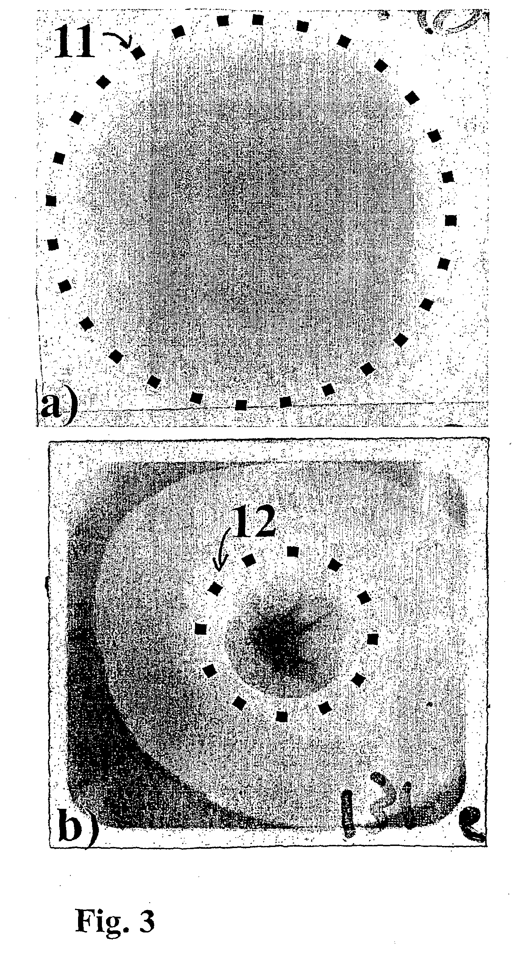

Figur 3 stellt die fokussierende Wirkung eines mit Laserpulsen beaufschlagten Hohlzylinders anhand zweier Aufnahmen dar. Dazu wurde in den Strahlengang ein "radiochromischer" Film eingebracht und mit dem Strahl beschossen. Ein solcher Film ändert seine Schwärzung bei Bestrahlung mehr oder weniger. Figur 3a zeigt das Profil des unfokussierten Protonenstrahls mit großer Divergenz11. Der Film ist in diesem Fall etwa 2 mm von der Folie 5 und damit von der Protonenquelle entfernt. Der Protonenstrahl nach 3b ist hingegen, wie oben beschrieben, mittels eines Hohlzylinders fokussiert ist und weist eine entsprechend kleine Divergenz 12 auf, was an dem wesentlich verkleinerten Strahlfleck deutlich zu erkennen ist. Der Film ist hier etwa 3,5 mm von der Folie 5 entfernt angeordnet.

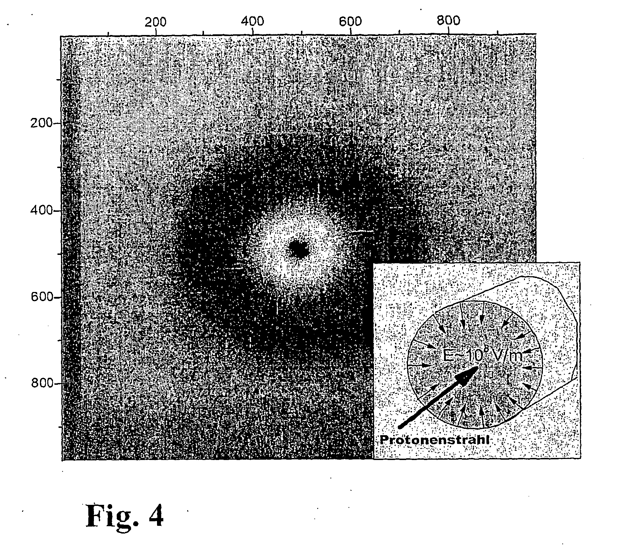

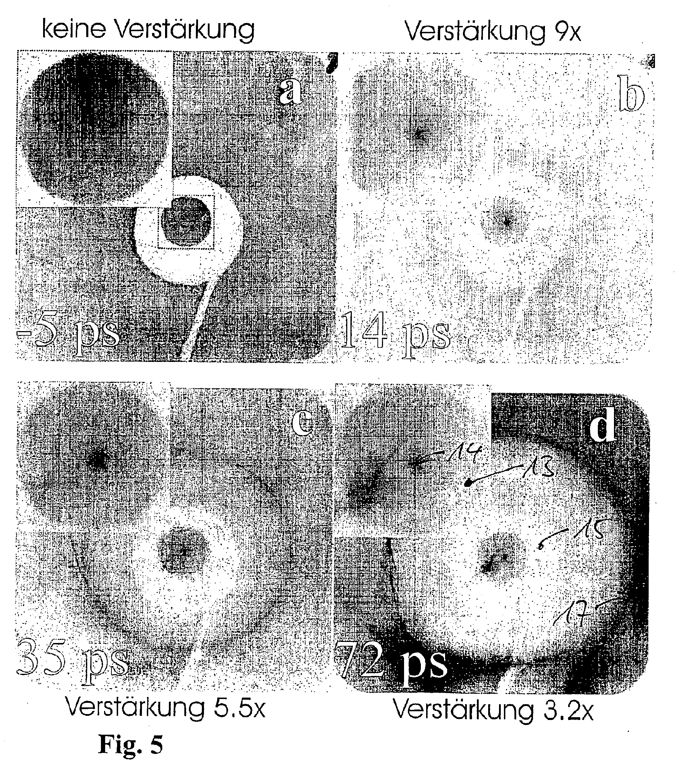

Der fokussierende Effekt kann in einer Simulation der einzelnen Partikelbahnen nachvollzogen werden (Figur 4). Dabei wurde ein radiales elektrisches Feld von 10⁸ V/m innerhalb des Zylinders angenommen. Die Simulation zeigt anhand der Graustufen eine Zunahme der Intensität im Zentrum des Protonenstrahls, der den hohlen, durch den Laserpuls aufgeladenen Zylinder verlässt. Die Maßangaben an den Achsen der Figur stellen die Strahlweite in Mikrometern dar. Der Einschub in der Figur 4 zeigt die angenommene Verteilung des elektrischen Feldes innerhalb des Zylinders. Dadurch werden die Protonen innerhalb des Zylinders in Richtung der Zylinderachse abgelenkt. Abhängig von der Länge des Zylinders führt das zu einer Kollimierung des Protonenstrahls auf der Achse, was im Experiment, wie in Figur 5 gezeigt wird, beobachtet werden kann.

Dabei wurde für die Messung nach Figur 5 folgender Versuchsaufbau realisiert: Der Zylinder hatte einen inneren Durchmesser von 650 µm und eine Wandstärke von 50 µm. Er bestand aus Dural (94% Al, 4% Cu, 1 % Mg) Er hatte eine Länge von 3mm. Die Eintrittsöffnung des Zylinders war 5 mm von der Folie 5 entfernt.

Um den kollimierten Protonenstrahl zu untersuchen, wurde im Abstand von etwa 4 cm hinter dem Hohlzylinder ein Stapel von mehreren radiochromischen Filmen angeordnet, auf den der kollimierte Protonenstrahl gerichtet wurde. Die Graustufen verdeutlichen die Intensität der Bestrahlung, wobei gilt: Je höher die Intensität, desto schwärzer die Färbung. Die Folien a) bis d) sind von hinten beginnend geordnet, wobei a) die hinterste Folie des Stapels war. Die Details zeigen jeweils das Innere des Zylinders. Bei a) ist zu erkennen, dass keine Fokussierung der besonders hochenergetischen Protonen stattgefunden hat, da der das Feld im Linsenkörper erzeugende Puls erst 5 ps nach dem Durchtritt dieser Protonen durch den Linsenkörper auf diesen auftraf. Insofern ist auch keine fokussierende Wirkung in Form einer Verstärkung zu erkennen. Auf der Folie b) hinterließen die Protonen eine Färbung, die 14 ps nach dem Auftreffen des felderzeugenden Laserpulses den Zylinder passierten. Deutlich zu erkennen ist der fokussierende Effekt, der eine Verstärkung um den Faktor neun im Verhältnis zum unfokussierten Strahl ausmacht. Eine Verstärkung von 5,5 ist bei den nach 35 ps auftreffenden Protonen zu erkennen (Folie b), während der Effekt bei den Protonen mit niedrigerer Energie, die nach 72 ps auf die erste Folie d) trafen, schon wieder abnimmt und nur noch mit einem Faktor 3,2 einhergeht. An Folie d) ist der Rand 13 des Hohlzylinders mit dem zentralen Fokus 14 deutlich zu erkennen. Der Bereich 15 geringer Intensität wird durch den Schatten des Hohlzylinders verursacht. Dieser grenzt an einen Bereich 16 an, der einem höheren Protonenfluss ausgesetzt war, die am Hohlzylinder vorbei gegangen ist.
Durch die Aufladung der Zylinderwand tritt eine sammelnde Wirkung außerhalb des Hohlzylinders ein, die durch den Ring 17 dargestellt ist.

## Patentansprüche

1. System zur Fokussierung und/oder zur Kollimierung eines Ionenstrahles (7), insbesondere eines Strahles beschleunigter Protonen, wobei das System eine Linse mit einem für den Ionenstrahl durchgängigen Linsenkörper (8) aufweist, wobei Mittel zur Erzeugung eines innerhalb des Linsenkörpers (8) sich ausbreitenden und den Ionenstrahl (7) fokussierenden, insbesondere elektrostatischen, Feldes vorgesehen sind, wobei der Linsenkörper (8) eine Wandung geringer Stärke aufweist,
**dadurch gekennzeichnet,**
**dass** die Mittel zur Felderzeugung eine Quelle für elektromagnetische. Strahlung umfassen, deren emittierter Stahl (9) auf die Außenseite der Wandung des Linsenkörpers (8) gerichtet ist,
wobei die Stärke der Wandung und die Qualität der elektromagnetischen Strahlung derart gewählt sind, dass die Strahlung freie Elektronen erzeugt, die aus der Wandung austreten und sich an der Austrittsseite der Wandung in einer Elektronenwolke sammeln.

2. System nach Anspruch 1,
**dadurch gekennzeichnet,**
**dass** die Wandung aus einem elektisch leitendem Material, insbesondere aus einer dünnen Metallfolie, gefertigt ist.

3. System nach Anspruch 1 oder 2,
**dadurch gekennzeichnet,**
**dass** der Linsenkörper als gestreckter, bezüglich seiner Achse symmetrischer Hohlkörper (8) ausgebildet ist, dessen Stirnseiten Öffnungen aufweisen, wobei die Öffnungen koaxial zur Symmetrieachse angeordnet sind.

4. System nach einem der vorherigen Ansprüche,
**dadurch gekennzeichnet,**
**dass** die Quelle für elektromagnetische Strahlung (9) ein gepulster Laser ist, wobei dieser derart ausgestaltet ist, dass seine Pulse eine Leistungsflächendichte von mehr als 10¹⁸ W/cm², insbesondere von 10¹⁹ W/cm², und eine Pulsdauer von kleiner als einigen Pikosekunden aufweisen.

5. System nach einem der vorherigen Ansprüche,
**gekennzeichnet durch**
eine Protonenquelle für einen in einer dünnen Folie (5) laserinduzierten Protonenstrahl (7), wobei die Folie (5) in Ausbreitungsrichtung des Strahles vor der Linse angeordnet ist.

6. System nach Anspruch 5, wenn bezogen auf Anspruch 4,
**dadurch gekennzeichnet,**
**dass** der für die Protonenquelle verwendete Laserstrahl (6) und der den Linsenkörper beaufschlagende Laserstrahl (9) Teile des Strahles eines einzigen Lasers sind.

7. System nach Anspruch 6,
**dadurch gekennzeichnet,**
**dass** der den Linsenkörper beaufschlagende Laserpuls (6) gegenüber dem für die Protonenquelle verwendeten Laserpuls (9) zeitlich verzögerbar ist, wobei die Verzögerung insbesondere einstellbar ist.

8. System nach einem der vorherigen Ansprüche,
**dadurch gekennzeichnet,**
**dass** die Wandung des Linsenkörpers (8) aus Metallfolie einer Stärke von weniger als 100 µm, insbesondere von weniger als 50 µm gefertigt ist.

9. System nach Anspruch 8,
**dadurch gekennzeichnet,**
**dass** die seitliche Wandung durchgängig geschlossen ist.

10. Verfahren zur Fokussierung eines Ionenstrahles (7) mittels einer in den Strahlengang eingebrachten Linse, wobei die Linse einen dünwandigen Linsenkörper (8) aufweist, der eine für den Ionenstrahl (7) durchgängige Öffnung umgibt, wobei innerhalb des Linsenkörpers (8) ein insbesondere elektrostatisches Feld erzeugt wird, das den durch die Öffnung hindurchtretenden Ionenstrahl (7) fokussiert,
**dadurch gekennzeichnet,**
**dass** das Feld durch äußere Beaufschlagung des dünnwandigen Linsenkörpers (8) mit elektromagnetischer Strahlung (9) hoher Energiedichte erzeugt wird.

11. Verfahren nach Anspruch 10,
**dadurch gekennzeichnet,**
**dass** das elektrostatische Feld mit einem gepulsten Laserstrahl (9) erzeugt wird.

12. Verfahren nach Anspruch 10 oder 11,
**dadurch gekennzeichnet,**
**dass** über den Grad der elektrischen Isolierung des Linsenkörpers (8) eine Selektivität der Fokussierung für Ionen einer bestimmten Energie erzeugt wird.

13. Verwendung des Systems nach einem der Ansprüche 1 bis 8 zur Fokussierung und/oder Kollimierung eines Strahles hochenergetischer Protonen, die mittels auf eine dünne Folie gerichteter Laserpulse erzeugt sind.

## Claims

1. A system for focusing and/or for collimating an ion beam (7), particularly a beam of accelerated protons, wherein the system has a lens with a lens body (8) continuous for ion beams, wherein means are provided for generating an electrostatic field, in particular, which propagates within the lens body (8) and focuses the ion beam (7), wherein the lens body (8) has a thin wall,
**characterised in that**,
the field-generating means consist of a source of electromagnetic radiation, from which the emitted beam (9) is directed at the outside of the wall of the lens body (8), wherein the thickness of the wall and the quality of the electromagnetic radiation are selected in such a manner that the radiation generates free electrons, which exit the wall and accumulate in a cloud of electrons on the exit side of the wall.

2. The system according to claim 1,
**characterised in that**,
the wall is made from an electrically conductive material, particularly a thin metal film.

3. The system according to claim 1 or 2,
**characterised in that**,
the lens body is in the form of an elongated hollow body (8) that is symmetrical to its axis and has openings in its front sides, wherein the openings are disposed coaxially to the symmetrical axis.

4. The system according to one of the preceding claims,
**characterised in that**,
the source of the electronic radiation (9) is a pulsed laser, wherein this is build in such a way that its pulses have an area output density of over 10¹⁸ W/cm², particularly 10¹⁹ W/cm², and a pulse duration of under a few picoseconds.

5. The system according to one of the preceding claims,
**characterised by**,
a proton source for a laser-induced proton beam in a thin film (5), wherein the film (5) is disposed in front of the lens in the propagation direction of the beam.

6. The system according to claim 5, when relating to claim 4,
**characterised in that**,
the laser beam (6) used for the proton source and the laser beam (9) impacting on the lens body are parts of the beam of a single laser.

7. The system according to claim 6,
**characterised in that**,
the laser pulse (6) impacting on the lens body can be delayed in time relative to the laser pulse (9) used for the proton source, wherein the delay is particularly adjustable.

8. The system according to one of the preceding claims,
**characterised in that**,
the walls of the lens body (8) are made from metal film with a thickness of under 100 µm, particularly under 50 µm.

9. The system according to claim 8,
**characterised in that**,
the side wall is continuously closed.

10. A method of focusing an ion beam (7) by means of a lens placed in the path of the beam, wherein the lens has a thin-walled lens body (8), which surrounds a continuous opening for the ion beam (7), wherein a particularly electrostatic field is generated within the lens body (8), which focuses the ion beam (7) emerging through the opening,
**characterised in that**,
the field is generated by the external impact of the thin-walled lens body (8) with electromagnetic radiation (9) with a high energy density.

11. The method according to claim 10,
**characterised in that**,
the electrostatic field is generated with a pulsed laser beam (9).

12. The method according to claim 10 or 11,
**characterised in that**,
selective focusing for ions of a particular energy is generated through the degree of electrical insulation on the lens body (8).

13. The use of the system according to one of the claims 1 to 8 for focusing and/or collimating a beam of high-energy protons, which are generated by means of laser pulses directed at a thin film.

## Revendications

1. Système de focalisation et/ou de collimation d'un faisceau ionique (7), en particulier d'un faisceau de protons accélérés, dans lequel le système présente une lentille avec un corps de lentille (8) passant pour le faisceau ionique, dans lequel des moyens pour générer un champ, en particulier un champ électrostatique, s'étendant à l'intérieur du corps de lentille (8) et focalisant le faisceau ionique (7) sont prévus, dans lequel le corps de lentille (8) présente une paroi de faible épaisseur,
**caractérisé en ce que**
les moyens de génération de champ comprennent une source pour un rayonnement électromagnétique dont le faisceau émis (9) est dirigé sur la face extérieure de la paroi du corps de lentille (8), dans lequel l'épaisseur de la paroi et la qualité du rayonnement électromagnétique sont sélectionnées de telle sorte que le rayonnement génère des électrons libres qui sortent de la paroi et se rassemblent du côté sortie de la paroi sous forme de nuage électronique.

2. Système selon la revendication 1, **caractérisé en ce que** la paroi est fabriquée à partir d'un matériau électriquement conducteur, en particulier à partir d'un film métallique mince.

3. Système selon la revendication 1 ou 2, **caractérisé en ce que** le corps de lentille est réalisé sous la forme d'un corps creux (8) allongé, symétrique par rapport à son axe, dont les faces frontales présentent des ouvertures, lesdites ouvertures étant disposées de façon coaxiale à l'axe de symétrie.

4. Système selon l'une quelconque des revendications précédentes, **caractérisé en ce que** la source de rayonnement électromagnétique (9) est un laser pulsé, ledit laser étant conçu de telle sorte que ses impulsions présentent une densité surfacique de puissance de plus de 10¹⁸ W/cm², en particulier de 10¹⁹ W/cm², et une durée d'impulsion inférieure à quelques picosecondes.

5. Système selon l'une quelconque des revendications précédentes, **caractérisé en ce qu'**une source de protons est disposée pour un faisceau de protons (7) produit par laser dans un film mince (5), le film (5) étant disposé dans la direction de propagation du faisceau devant la lentille.

6. Système selon la revendication 5, lorsqu'elle fait référence à la revendication 4, **caractérisé en ce que** le faisceau laser (6) utilisé pour la source de protons et le faisceau laser (9) sollicitant le corps de lentille font partie du faisceau d'un seul laser.

7. Système selon la revendication 6, **caractérisé en ce que** l'impulsion laser (6) sollicitant le corps de lentille peut être retardée dans le temps par rapport à l'impulsion laser (9) utilisée pour la source de protons, le retard étant en particulier réglable.

8. Système selon l'une quelconque des revendications précédentes, **caractérisé en ce que** la paroi du corps de lentille (8) est fabriquée à partir d'un film métallique d'une épaisseur inférieure à 100 µm, en particulier inférieure à 50 µm.

9. Système selon la revendication 8, **caractérisé en ce que** la paroi latérale est fermée de façon passante.

10. Procédé de focalisation d'un faisceau ionique (7) au moyen d'une lentille introduite sur la trajectoire du faisceau, dans lequel la lentille présente un corps de lentille (8) à paroi mince qui entoure une ouverture passante pour le faisceau ionique (7), dans lequel à l'intérieur du corps de lentille (8) un champ en particulier électrostatique est généré qui focalise le faisceau ionique (7) passant par l'ouverture,
**caractérisé en ce que**
le champ est généré par une sollicitation extérieure du corps de lentille (8) à paroi mince avec un rayonnement électromagnétique (9) de haute densité énergétique.

11. Procédé selon la revendication 10, **caractérisé en ce que** le champ électrostatique est généré par un faisceau laser pulsé (9).

12. Procédé selon les revendications 10 ou 11, **caractérisé en ce que** le degré d'isolation électrique du corps de lentille (8) génère une sélectivité de la focalisation pour des ions d'une certaine énergie.

13. Utilisation du système selon l'une quelconque des revendications 1 à 8, pour la focalisation et/ou la collimation d'un faisceau de protons à grande énergie qui sont générés au moyen d'impulsions laser dirigées sur un film mince.
